# EUROPEAN PATENT APPLICATION

(11) **EP 4 104 836 A1**
(43) Date of publication of application: **21.12.2022**
(21) Application number: 21766099.2
(22) Date of filing: 28.06.2021
(51) Int. Cl.: A61K 31/496, A61K 45/06, A61P 27/02, A23L 33/10

(54) **PHARMACEUTICAL COMPOSITION FOR TREATING MACULAR DEGENERATION, CONTAINING IMIDAZOLINE DERIVATIVE COMPOUND AS ACTIVE INGREDIENT**

(30) Priority: 04.05.2021 KR 20210058106
(71) Applicant: Fusion Biotechnology Co., Ltd., Daejeon 34051 (KR)
(72) Inventor: KIM, Chaekyu, Nam-gu Ulsan 44707 (KR)
(74) Representative: Vossius & Partner Patentanwälte Rechtsanwälte mbB
(86) International application number: PCT/KR2021/008074
(87) International publication number: WO 2022/234888

(57) **Abstract**

Provided are a pharmaceutical composition for treating macular degeneration, including an imidazoline derivative compound as an active ingredient. When the pharmaceutical composition including the imidazoline derivative compound or the pharmaceutically acceptable salt thereof as an active ingredient is used, senescent cells are selectively killed and thus drusen and macular atrophy are reduced, and thus the composition can be useful for preventing and/or treating macular degeneration.

## Description

### TECHNICAL FIELD

The present disclosure relates to a pharmaceutical composition for treating macular degeneration including an imidazoline derivative compound as an active ingredient.

### BACKGROUND ART

Normal vision occurs when light enters the front of the eye, which focuses on very sensitive and weak cells called photoreceptors, as a lens, that are arranged inside at the back of the eye. The photoreceptors, along with other neurons, form the retina of the eye. The macula is a part of the retina located near the posterior center of the eye, including the fovea responsible for a clear central vision. The nerve tissue located in the center of the retina inside the eye is called the macula. Macula plays a very important role in vision, since most of the visual cells responding to light stimulation are gathered here, and the place where the image of the object is formed is also the center of the macula.

The retina may suffer from various diseases such as retinal inflammation, macular degeneration, diabetic retinopathy, macular edema, retinal damage, glaucoma, optic neuropathy, retinal vascular diseases, ocular vascular diseases, glaucoma, and the like due to various causes, and in particular, eye diseases that cause vision disorders due to degeneration by various causes in the macula part (retinal pigment epithelium, Bruch's membrane, and choroidal capillary complexes) are referred to as macular degeneration (disorder), and macular degeneration is known as one of three major blindness diseases together with glaucoma and diabetic retinal diseases. Such macular degeneration affects the central vision and makes the middle of the field of vision blurred, and may cause difficulties in carrying out sophisticated activities such as reading and driving due to central dark spots, metamorphosis (a symptom in which things are distorted), or loss of vision of a local part, and in severe cases, the macular degeneration leads to a real name.

The most representative cause of macular degeneration is an increase in age, and it is known that macular degeneration is somewhat related to family history, race, smoking, etc. In particular, senile (degenerative) age-related macular degeneration (AMD) has already become the most common disease in Western societies that causes blindness among seniors aged 55 or older, and in the United States, the number of patients already ranks first in the list of elderly blindness, and as the aging population progresses rapidly in Korea, the number of patients is increasing. This is assumably due to westernized eating habits and the increase in UV exposure caused by the destruction of the ozone layer. In particular, macular degeneration was originally a disease mainly caused by the elderly, but recently, the age group thereof has been lowered from the elderly in their 60s to the middle-aged in their 40s and 50s in Korea, and the incidence rate of the middle-aged has also been rapidly increasing in recent years.

Macular degeneration can be largely classified into two types, dry and wet, and about 90% of macular degeneration patients are dry macular degeneration patients. One form of macular degeneration, dry macular degeneration, is classified as atrophic (non-exudative) and is called geographical atrophy, and is an early-stage disease in which drusen is deposited on the retina at a subretinal level. Such deposition of drusen may cause aging or thinning of macular tissue. As a result of such drusen deposition, damage to the central vision can gradually occur. Frequently, senile macular degeneration occurs with dry macular degeneration. In the case of a dry form macular degeneration, it is difficult to perform self-diagnosis because there is no sign in the early stages and the risk of blindness is relatively low due to slow progress, but the need for prevention is further emphasized because dry macular degeneration has no established treatment yet.

### PRIOR ART DOCUMENTS

### PATENT DOCUMENTS

(Patent Document 1) Korean Registered Patent No. 10-2050506

### DESCRIPTION OF EMBODIMENTS

### TECHNICAL PROBLEM

One aspect provides a pharmaceutical composition for preventing or treating macular degeneration including an imidazoline derivative compound represented by Formula 1 or a pharmaceutically acceptable salt thereof as an active ingredient: 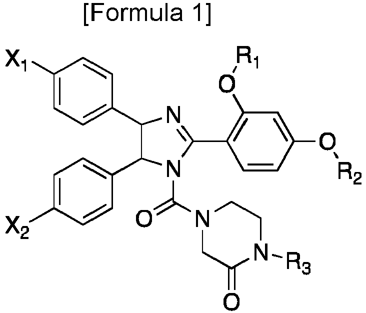 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

Another aspect of the present disclosure provides a pharmaceutical composition for preventing or treating macular degeneration, the pharmaceutical composition including the imidazoline derivative compound or a pharmaceutically acceptable salt thereof as an active ingredient, wherein the imidazoline derivative compound is co-administered with an antioxidant.

Another aspect provides a health functional food for preventing or ameliorating macular degeneration, the health functional food including the imidazoline derivative compound represented by Formula 1 or a salt thereof as an active ingredient.

Another aspect provides a method of preventing or treating macular degeneration, including administering an imidazoline derivative compound represented by Formula 1 or a pharmaceutically acceptable salt thereof to a subject in need thereof: 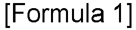 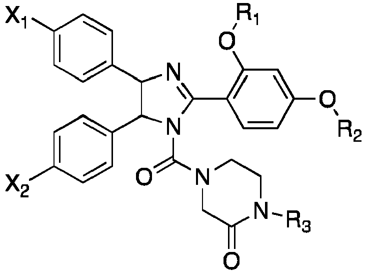 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

Another aspect provides the use of an imidazoline derivative compound represented by Formula 1 or a pharmaceutically acceptable salt thereof for the manufacture of a medicament for the prevention or treatment of macular degeneration: 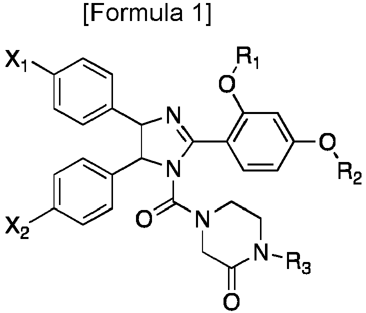 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

### TECHNICAL SOLUTION TO PROBLEM

One aspect provides a pharmaceutical composition for preventing or treating macular degeneration including an imidazoline derivative compound represented by Formula 1 or a pharmaceutically acceptable salt thereof as an active ingredient: 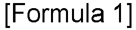 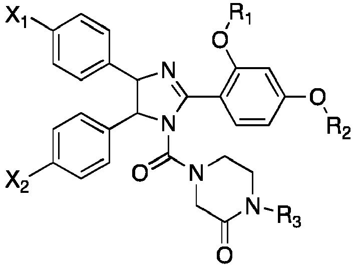 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

The imidazoline derivative compounds may be optionally substituted with one or more substituents, such as those presented herein as an example of certain classes, subclasses, and species of the present disclosure.

The term "alkyl" used herein refers to a saturated aliphatic hydrocarbon group including 1-10 (for example, 1-8, 1-6, or 1-4) carbon atoms. The alkyl group may be linear or branched. Examples of alkyl groups include, but are not limited to, methyl, ethyl, propyl, isopropyl, butyl, isobutyl, sec-butyl, tert-butyl, n-pentyl, n-heptyl, and 2-ethylhexyl. The alkyl group may be substituted (that is, optionally substituted) with one or more substituents selected from halo, phosphor, a cycloaliphatic group [for example, cycloalkyl, or cycloalkenyl], a heterocycloaliphatic group [for example, heterocycloalkyl, or heterocycloalkenyl], aryl, heteroaryl, alkoxy, aroyl, heteroaroyl, acyl [for example, (aliphatic)carbonyl, (cycloaliphatic)carbonyl, or (heterocycloaliphatic)carbonyl], nitro, cyano, amido [for example, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [for example, aliphatic amino, cycloaliphatic amino, or heterocycloaliphatic amino], sulfonyl [for example, aliphatic-SO₂-], sulfinyl, sulfanyl, sulfoxy, urea, thiourea, sulfamoyl, sulfamide, oxo, carboxy, carbamoyl, cycloaliphatic, heterocycloaliphaticoxy, aryloxy, heteroaryloxy, aralkyloxy, heteroarylalkoxy, alkoxycarbonyl, alkylcarbonyloxy, and hydroxy. Some examples of substituted alkyl include, without limitation, carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (for example (alkyl-SO₂-amino)alkyl), aminoalkyl, amidoalkyl, (cycloaliphatic)alkyl, haloalkyl, an amine cation, quaternary ammonium, and quaternary phosphonium.

The term "alkenyl" used herein refers to an aliphatic carbon group including 2-10 (for example, 2-8, 2-6, or 2-4) carbon atoms and at least one double bond. Like alkyl groups, alkenyl groups may be straight or branched. Examples of alkenyl groups include allyl, isoprenyl, 2-butenyl, and 2-hexenyl. The alkenyl group may be optionally substituted with one or more substituents selected from halo, phosphor, a cycloaliphatic group [for example, cycloalkyl, or cycloalkenyl], a heterocycloaliphatic group [for example, heterocycloalkyl, or heterocycloalkenyl], aryl, heteroaryl, alkoxy, aroyl, heteroaroyl, acyl [for example, (aliphatic)carbonyl, (cycloaliphatic)carbonyl, or (heterocycloaliphatic)carbonyl], nitro, cyano, amido [for example, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [for example, aliphatic amino, cycloaliphatic amino, heterocycloaliphatic amino, or aliphatic sulfonyl amino], sulfonyl [for example, alkyl-SO₂-, cycloalipathic -SO₂-, or aryl-SO₂-], sulfinyl, sulfanyl, sulfoxy, urea, thiourea, sulfamoyl, sulfamide, oxo, carboxy, carbamoyl, cycloaliphatic, heterocycloaliphaticoxy, aryloxy,heteroaryloxy, aralkyloxy, heteroaralkoxy, alkoxycarbonyl, alkylcarbonyloxy, and hydroxy. Examples of substituted alkenyls include, without limitation, cyanoalkenyl, alkoxyalkenyl, acylalkenyl, hydroxyalkenyl, aralkenyl, (alkoxyaryl)alkenyl, (sulfonylamino)alkenyl (for example (alkyl-SO₂-amino)alkenyl), aminoalkenyl, amidoalkenyl, (cycloaliphatic)alkenyl, and haloalkenyl.

The term "alkynyl" used herein refers to an aliphatic carbon group including 2-10 (for example, 2-8, 2-6, or 2-4) carbon atoms and at least one triple bond. The alkynyl group may be linear or branched. Examples of alkynyl groups include, but are not limited to, prophargyl and butynyl. The alkynyl group may be optionally substituted with at least one substituent selected from aroyl, heteroaroyl, alkoxy, cycloalkyloxy, heterocycloalkyloxy, aryloxy, heteroaryloxy, aralkyloxy, nitro, carboxy, cyano, halo, hydroxy, sulfo, mercapto, sulfanyl [for example, aliphatic sulfanyl or cycloaliphatic sulfanyl], sulfinyl [for example, aliphatic sulfinyl or cycloaliphatic sulfinyl], sulfonyl [for example, aliphatic-SO₂-, aliphaticamino-SO₂-, or cycloaliphatic-SO₂-], amido [for example, aminocarbonyl, alkylaminocarbonyl, alkylcarbonylamino, cycloalkylaminocarbonyl, heterocycloalkylaminocarbonyl, cycloalkylcarbonylamino, arylaminocarbonyl, arylcarbonylamino, aralkylcarbonylamino, (heterocycloalkyl)carbonylamino, (cycloalkylalkyl)carbonylamino, heteroaralkylcarbonylamino, heteroarylcarbonylamino, or heteroarylaminocarbonyl], urea, thiourea, sulfamoyl, sulfamide, alkoxycarbonyl, alkylcarbonyloxy, a cycloaliphatic group, a hetero cycloaliphatic group, aryl, heteroaryl, acyl [for example, (cycloaliphatic)carbonyl or (heterocycloaliphatic)carbonyl], amino [for example, aliphatic amino], sulphoxy, oxo, carboxy, carbamoyl, (cycloaliphatic)oxy, (heterocycloaliphatic)oxy, and (heteroaryl)alkoxy.

The term "alkoxy" used herein refers to a group in which a hydrogen atom of hydroxyl is substituted with alkyl, alkenyl, or alkynyl. Suitable alkoxy include, but are not limited to, a C₁ to C₁₀ alkoxy such as methoxy, ethoxy, propoxy, butoxy and pentoxy.

The term "halogen" used herein refers to fluorine, chlorine, bromine, or iodine.

The compounds of the present disclosure may be optionally substituted with, for example, one or more substituents presented herein as an example as certain classes, subclasses and species of the disclosure. Unless stated otherwise, R₁ to R₃ of the disclosure may be optionally substituted with one or more substituents described herein. Each substituent of a particular group may be further optionally substituted with one to three groups of halo, cyano, oxo, alkoxy, hydroxy, amino, nitro, aryl, cycloaliphatic, heterocycloaliphatic, heteroaryl, haloalkyl, and alkyl. For example, the alkyl group may be optionally substituted with alkylsulfanyl, and the alkylsulfanyl may be optionally substituted with one to three groups of halo, cyano, oxo, alkoxy, hydroxy, amino, nitro, aryl, haloalkyl, and alkyl. As another example, the cycloalkyl moiety of (cycloalkyl)carbonylamino may be optionally substituted with one to three groups of halo, cyano, alkoxy, hydroxy, nitro, haloalkyl, and alkyl. When two alkoxy groups are bonded to the same atom or adjacent atoms, the two alkoxy groups may form a ring with the atom(s) to which they are bonded.

In some embodiments, in Formula 1, R₁ may be isopropyl, R₂ may be methyl, R₃ may be hydrogen, and X₁ and X₂ may each be Cl.

In an embodiment, the imidazoline derivative compound may be Nutlin-1, Nutlin-2, or Nutlin-3, and Nutlin-3 may be Nutlin-3a or Nutlin-3b.

In an embodiment, the composition may further include an antioxidant, and the antioxidant may include at least one selected from nicotinamide, anthocyanin, benzenediol abietane diterpene, carnosine, carotenoid, xanthophyll, carotenoid in saffran, curcuminoid, cyclopentenone prostaglandin, flavonoid, prenylflavonoid, retinoid, stilbenoid, uric acid, vitamin A, vitamin B1, vitamin B2, vitamin B3, vitamin B6, vitamin B9, vitamin B12, vitamin C, vitamin E, selenium, zinc, an inhibitor and scavenger of lipid peroxidation and by-products thereof, tirilazad, and analogs, derivatives, salts, and combinations thereof.

In an embodiment, the imidazoline derivative compound may overexpress at least one of p53 and p21 in senescent cells, and thus may selectively kill senescent cells.

In an embodiment, the macular degeneration may be at least one selected from age-related macular degeneration (AMD), wet macular degeneration, and dry macular degeneration. A case where lesions such as drusen (a condition where waste materials are piled up in the macular part) or atrophy of retinal pigment epithelium occur in the retina is called dry macular degeneration, and a disease caused by the growth of choroidal neovessels under the retina is called wet macular degeneration. When the imidazoline derivative compound according to the present disclosure is administered or the combination of the imidazoline derivative compound and the antioxidant is administered to the aged retina, drusen is reduced and Bruch's membrane becomes thin, indicating that the administration thereof is effective for dry macular degeneration. According to an embodiment, when Nutlin-3 is administered alone, or Nutlin-3 and nicotinamide are administered in combination, it can be seen that drusen is reduced and Bruch's membrane becomes thin.

Another aspect of the present disclosure provides a pharmaceutical composition for preventing or treating macular degeneration, the pharmaceutical composition including an imidazoline derivative compound represented by Formula 1 or a pharmaceutically acceptable salt thereof as an active ingredient, wherein the imidazoline derivative compound is co-administered with an antioxidant: 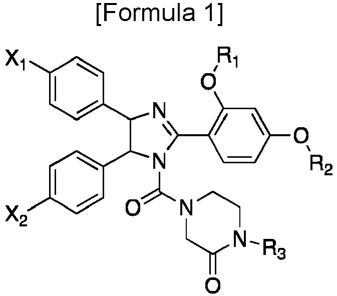 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

Another aspect provides a method of preventing or treating macular degeneration, including administering an imidazoline derivative compound represented by Formula 1 or a pharmaceutically acceptable salt thereof to a subject in need thereof: 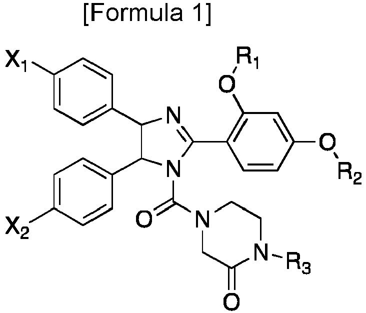 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

The co-administration may indicate that the imidazoline derivative compound and the antioxidant are administered simultaneously, sequentially or individually and in any order.
In detail, the co-administration may be performed by simultaneously administering the imidazoline derivative compound and the antioxidant, or by administering any one of the imidazoline derivative compound and the antioxidant and then the other one. The combination therapy according to the present disclosure may be defined as being capable of providing a synergistic effect if the efficacy measured through, for example, the degree of reaction, the rate of reaction, the disease progression duration, or the survival period is therapeutically superior to the efficacy obtained by administering one or the remainder of the components of the combination therapy at a normal dose. For example, when the efficacy thereof is therapeutically superior to the efficacy obtained using each of the components, the efficacy of the combination therapy is synergistic. In particular, it is considered that there is a synergistic effect when the conventional dose of the imidazoline derivative compound is reduced while the side effects are reduced/reduced or small, compared to when each component is used at a conventional dose, without negatively affecting at least one of the degree of reaction, the rate of reaction, the disease progression duration, and survival data, for example, the response duration. When the imidazoline derivative compound and the antioxidant are co-administered, the imidazoline derivative compound or a pharmaceutically acceptable salt thereof may be administered in an amount of about 1 mg/kg to about 100 mg/kg, for example, 10 mg/kg to 80 mg/kg, 10 mg/kg to 60 mg/kg, 20 mg/kg to 80 mg/kg, 20 mg/kg to 60 mg/kg, 30 mg/kg to 80 mg/kg, 30 mg/kg to 60 mg/kg, 40 mg/kg to 80 mg/kg, or 40 mg/kg to 60 mg/kg. In addition, the antioxidant may be administered in an amount of about 50 mg/kg to 150 mg/kg, for example, 60 mg/kg to 150 mg/kg, 60 mg/kg to 130 mg/kg, 60 mg/kg to 110 mg/kg, 70 mg/kg to 150 mg/kg, 70 mg/kg to 130 mg/kg, 70 mg/kg to 110 mg/kg, 80 mg/kg to 150 mg/kg, 80 mg/kg to 130 mg/kg, 80 mg/kg to 110 mg/kg, 90 mg/kg to 150 mg/kg, 90 mg/kg to 130 mg/kg, or 90 mg/kg to 110 mg/kg.

The compound may be present in the form of a pharmaceutically acceptable salt. The pharmaceutically acceptable salt includes an addition salt of an acid or a base and a stereochemical isomer form thereof, and may be, for example, an addition salt of an organic acid or an inorganic acid. The salt may include any salt that maintains the activity of a parent compound in a subject to be administered and does not cause an undesirable effect, and is not particularly limited.

Such salts include an inorganic salt and an organic salt, and may be, for example, acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, lactic acid, bicarbonic acid, bisulfuric acid, bitartaric acid, oxalic acid, butyric acid, calcium idet, carbonic acid, chlorobenzoic acid, citric acid, idetic acid, toluenesulfonic acid, fumaric acid, gluceptic acid, essylic acid, pamoic acid, gluconic acid, methylnitric acid, malonic acid, hydrochloric acid, hydroiodoic acid, hydroxynaphthoic acid, isethionic acid, lactobionic acid, mandelic acid, mucic acid, naphthylic acid, muconic acid, p-nitromethanesulphonic acid, hexamic acid, pantothenic acid, monohydrogenphosphoric acid, dihydrogen phosphoric acid, salicylic acid, sulfamic acid, sulfanilic acid, or methanesulfonic acid.

Further, the salts include salts of alkaline and alkaline earth metals such as ammonium salts, lithium salts, sodium salts, potassium salts, magnesium salts, and calcium salts, for example, salts having organic bases such as benzathine, N-methyl-D-glucamine, or hydrabamine salts, and salts having amino acids such as arginine, or lysine. In addition, the salt form may be converted into a free form by treatment with an appropriate base or acid.

The term "therapeutic agent" or "pharmaceutical composition" refers to a molecule or compound that confers some beneficial effects upon administration to a subject. The beneficial effects may include: making diagnostic decisions possible; amelioration of disease, a symptom, a disorder, or a condition; and reduction or prevention of the onset of a disease, symptom, disorder, or disease; and generally, a response to disease, a symptom, a disorder, or a condition.

The pharmaceutical composition of the present disclosure may be in any form suitable for the intended administration method. Regarding the pharmaceutical composition of the present disclosure, "administration" refers to introducing a certain material into a patient by any suitable method, and the administration route of the pharmaceutical composition may be any general route as long as the drug can reach the target tissue. For example, the administration route may be topical ocular administration (for example, the surrounding of eyeball, such as subTenon's),subconjunctival, intraocular, intravitreal, interior chamber, subretinal, suprachoroidal and posterior chamber administration), intraperitoneal administration, intravenous administration, intramuscular administration, subcutaneous administration, intradermal administration, oral administration, topical administration, intranasal administration, intrapulmonary administration, intrarectal administration, and the like, but is not limited thereto. In addition, the pharmaceutical composition of the present disclosure may be administered by any device that allows the active ingredient to be transferred to a target cell. The route of administration of the pharmaceutical composition of the present disclosure may vary depending on the type of disease to be applied.

The pharmaceutical composition provided in the present specification may be formulated into an oral dosage form such as powder, a granule, a tablet, a capsule, a suspension, an emulsion, a syrup, an aerosol, and the like, or an parenteral dosage form such as a suspension, an emulsion, a lyophilized preparation, an external preparation, a suppository, a sterile injection solution, an implantable preparation, and the like, formulated according to a conventional method.

The pharmaceutical composition may further include a pharmaceutically acceptable excipient usable in formulation in addition to the active ingredient (that is, the compound or the pharmaceutically acceptable salt thereof).

Excipients that can be used in the formulation of the pharmaceutical compositions of the present disclosure include carriers, vehicles, diluents, solvents such as monohydric alcohols, for example, ethanol or isopropanol, and polyhydric alcohols, for example, glycerol and edible oils such as soybean oil, coconut oil, olive oil, safflower oil, cottonseed oil, or oily esters such as ethyl oleate, isopropyl myristate; a binder, an adjuvant, a solubilizer, a thickener, a stabilizer, a disintegrant, a glydent, a lubricant, a buffer, an emulsifier, a wetting agent, a suspension, a sweetening agent, a coloring agent, a flavoring agent, a coating agent, a preservative, an antioxidant, a processing agent, a drug delivery modifier and enhancer, for example, calcium phosphate, magnesium state, talc, monosaccharide, disaccharide, starch, gelatin, cellulose, methylcellulose, sodium carboxymethyl cellulose, dextrose, hydroxypropyl-β-cyclodextrin, polyvinylpyrrolidone, low melting point wax, ion exchange resin, and the like, but the present disclosure is not limited thereto.

The pharmaceutically acceptable carrier included in the pharmaceutical composition of the present disclosure may be any carrier that is commonly used in preparations, and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but is not limited thereto. The pharmaceutical composition of the present disclosure may further include, in addition to these components, a lubricant, a wetting agent, a sweetening agent, a flavoring agent, an emulsifier, a suspending agent, a preservative, and the like. Suitable pharmaceutically acceptable carriers and preparations are described in detail in Remington's Pharmaceutical Sciences (19th ed., 1995).

The pharmaceutical composition may be formulated in the form of various oral dosage forms. For example, the formulation can be any oral dosage form, such as tablets, pills, hard and soft capsules, liquids, suspensions, emulsifiers, syrups, granules, elixirs, and the like. Such formulations for oral administration may include, in addition to the active ingredient, a pharmaceutically acceptable carrier, for example, a diluent such as lactose, dextrose, sucrose, mannitol, sorbitol, cellulose and/or glycine; or a glydent such as silica, talc, stearic acid and magnesium or calcium salts thereof and/or polyethylene glycol, in accordance with the conventional structure of each formulation.

In addition, in a case where the formulation for oral administration is a tablet, the formulation may include a binder such as magnesium aluminum silicate, starch paste, gelatin, tragacanth, methylcellulose, sodium carboxymethylcellulose, and/or polyvinyl pyrrolidine, and in some cases, the formulation may include a disintegrating agent such as starch, agar, and alginic acid or a sodium salt thereof, a boiling mixture and/or an absorbent, a coloring agent, a flavoring agent, a sweetening agent, and the like.

In addition, the pharmaceutical composition may be formulated in the form of a parenteral dosage form, and in this case, subcutaneous injection, intravenous injection, intramuscular injection, or intrathoracic injection may be used. In this regard, in order to be formulated into the parenteral dosage form, the pharmaceutical composition may be prepared by mixing an active ingredient with a stabilizer or a buffer in water to prepare a solution or a suspension, and the solution or the suspension may be prepared as a unit dosage form of an ampoule or a vial.

In addition, the pharmaceutical composition may be sterilized, or may further include a supplement such as a preservative, a stabilizer, a hydrating agent, or an emulsifying accelerator, a salt for controlling osmotic pressure, and/or a buffer, or may further include other therapeutically useful materials, and may be formulated according to a conventional method of mixing, granulating, or coating.

The amount of the compound or the pharmaceutically acceptable salt thereof in the pharmaceutical composition may be appropriately controlled according to the purpose of use of the pharmaceutical composition, the form of the formulation, and the like, and may be, for example, from 0.001 wt% to 99 wt%, from 0.001 wt% to 90 wt%, from 0.001 wt% to 50 wt%, from 0.01 to 50 wt%, from 0.1 wt% to 50 wt%, or from 1 wt% to 50 wt% based on the total weight of the pharmaceutical composition, but is not limited thereto.

In addition, the therapeutically effective amount of the compound or the pharmaceutically acceptable salt thereof included in the pharmaceutical composition of the present disclosure refers to an amount required for administration in order to expect a disease treatment effect. Accordingly, the amount may be adjusted according to the type of the patient's disease, the severity of the disease, the type of the active ingredient to be administered, the type of the formulation, the age, sex, weight, health condition, diet of the patient, the time of administration of the drug, and the method of administration. For example, to a mammal including a human, the pharmaceutically effective amount of 0.01 mg/kg to 500 mg/kg (body weight) may be administered per day. The pharmaceutically effective amount may vary based on an amount capable of obtaining a desired effect, for example, an effect of treating and/or preventing aging-related diseases. Via oral or parenteral routes (for example, eye injection, intravenous injection, muscle injection, etc.), the pharmaceutically effective amount may be administered once per day, or may be divided into two or more dosages, which are then administered separately.

Due to including of the administration of the pharmaceutical composition to the subject in an amount effective to prevent or treat macular degeneration, macular degeneration of the subject can be treated.

The subject may be a mammal. The mammal may be a human, a dog, a cat, cow, a goat, or a pig.

The terms "treatment" or "treat" or "alleviate" or "ameliorate" are used herein interchangeably. These terms include therapeutic benefits and/or prophylactic benefits, but are not limited thereto. For example, these terms may indicate favorable or desired results. Therapeutic benefits refer to any therapeutically significant improvement in or effect on one or more diseases, disorders or symptoms under treatment. Regarding prophylactic benefits, the composition may be administered to a subject at risk of developing a particular disease, disorder, or symptom, or to a subject reporting one or more physiological symptoms of the disease, although the disease, disease, or symptom does not yet appear.

The term "effective amount" or "therapeutically effective amount" refers to an amount of agent sufficient to cause favorable or desired results. The therapeutically effective amount may vary depending on one or more of the subject and condition being treated, the weight and age of the subject, the severity of the condition, the mode of administration, and the like, which may be readily determined by those skilled in the art. Furthermore, the term applies to a capacity that is to provide an image for detection obtained by any of the imaging methods described herein. The particular dose may vary depending on one or more of the particular agent selected, the following dosing regimen, whether it is administered in combination with another compound, the timing of administration, the tissue being imaged, and the body delivery system carrying the same.

Another aspect provides a health functional food for preventing or ameliorating macular degeneration, the health functional food including the imidazoline derivative compound represented by Formula 1 or a salt thereof as an active ingredient: 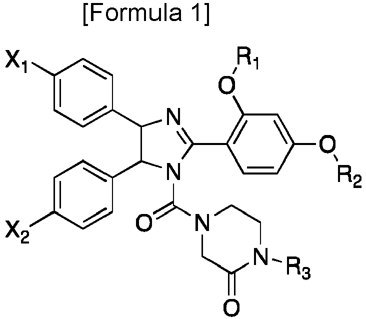 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

In an embodiment, the health functional food may further include an antioxidant.

The term "health functional food" used herein refers to a food produced and processed using an ingredient or component which has a function useful for the human body according to Act No. 6727 of the Health Functional Foods, and refers to a product that is ingested to obtain effects useful for the health, such as the control of nutrients for the structure and function of the human body and physiological effects.

When the composition of the present disclosure is used as a food additive, the composition may be added as it is or used together with other foods or components, and may be appropriately used according to a conventional method. The mixing amount of the active ingredient may be appropriately determined according to the purpose of use. The food additive usually indicates a component that is intentionally added to food in a small amount for the purpose of improving appearance, flavor, tissue, or storability, and is used to improve preservation or the palatability by improving the quality of foods, and improve nutritional values and the actual value of food. As defined in Article 2, paragraph 2 of the Food Sanitation Act, the food additive may be a material that is used in addition, mixing, infiltration, or other methods in manufacturing or preserving food.

The food of the present disclosure is not particularly limited. Examples of foods to which the composition of the present disclosure can be added, include meat, sausages, bread, chocolate, candies, snacks, confectionery, instant noodles, other kinds of noodles, gums, dairy products including ice cream, various soups, beverages, tea, drinks, alcoholic beverages, vitamin complexes, and the like. The foods used herein may include any food that is considered as a food in a general sense, and includes foods used as feed for animals.

The health functional food of the present disclosure may include a conventional food additive, and whether the food additive is suitable or not is determined according to the specifications and standards for the relevant item in accordance with the general rules and the general test methods of the approved food additive procedure of the Korea Food and Drug Administration, unless provided otherwise.

In addition, the food composition of the present disclosure may include various nutrients, vitamins, electrolytes, flavoring agents, coloring agents, pectic acid and salts thereof, alginic acid and salts thereof, organic acids, protective colloid thickeners, pH adjusters, stabilizers, preservatives, glycerin, alcohols, carbonating agents used in carbonated beverages, and the like. In addition, the food composition of the present disclosure may include flesh for the preparation of natural fruit juices, fruit juice beverages, and vegetable beverages.

In addition, the food may be prepared in formulations such as tablets, granules, powders, capsules, liquid solutions, and pills according to known preparation methods. There is no particular limitation in other components except for including the compound of the present disclosure as an active ingredient, and various conventional flavoring agents or natural carbohydrates may be contained as additional components.

For example, in the case of the health functional food in the form of tablets, a mixture including the compound represented by Formula 1, which is an active ingredient of the present disclosure, and an excipient, a binder, a disintegrant, and other additives, is granulated by a conventional method, and then a glydent is added thereto, followed by compression-molding, or the mixture may be directly compression-molded. In addition, the tablet-type health functional food may contain a flavoring agent or the like, if needed.

Among capsule-type health functional foods, a hard capsule may be prepared by filling conventional hard capsules with a mixture of a compound of Formula 1, which is an active ingredient of the present disclosure, and an additive such as an excipient, and a soft capsule may be prepared by filling a capsule base such as gelatin with a mixture of a compound of Formula 1 and an additive such as an excipient. The soft capsule may contain a plasticizer such as glycerin or sorbitol, a coloring agent, a preservative, and the like, when needed.

The ring-shaped health functional food may be prepared by molding a mixture including the compound of Formula 1, which is an active ingredient of the present disclosure, and an excipient, a binder, a disintegrant, and the like according to a known method, and if needed, the resultant product may be removed with a white sugar or other coating materials as necessary, or may be surface-coated with a material such as starch or talc.

The health functional food in a granular form may be prepared in a granular form by a known method by mixing the compound of Formula 1, which is an active ingredient of the present disclosure, with an excipient, a binder, a disintegrant, and the like, and may contain a flavoring agent, a flavoring agent, and the like, if necessary.

The terms and methods described above are equally applied among the disclosures.

### ADVANTAGEOUS EFFECTS OF DISCLOSURE

The composition including the imidazoline derivative compound or the pharmaceutically acceptable salt thereof as an active ingredient according to one aspect of the present disclosure has the effect of being useful for preventing and/or treating macular degeneration by selectively killing senescent cells to reduce drusen and macular atrophy.

### BRIEF DESCRIPTION OF DRAWINGS

FIG. 1 shows an image by which the induction of cells into senescent cells when ARPE-19 cells were treated with doxorubicin, was confirmed by SA-β-gal staining.
FIG. 2 shows a graph of the number of cells by which the induction of the cells into senescent cells when ARPE-19 cells were treated with doxorubicin, was confirmed by SA-β-gal staining.
FIG. 3 shows an image and a graph by which high p53 expression in senescent cells induced by treating ARPE-19 cells with doxorubicin, was confirmed.
FIG. 4 shows an image and a graph by which high p21 expression in senescent cells induced by treating ARPE-19 cells with doxorubicin, was confirmed.
FIG. 5 shows a graph by which high expression of inflammatory factors in senescent cells induced by treating ARPE-19 cells with doxorubicin, was confirmed.
FIG. 6 shows a graph by which the senescent cell death in the case of the treatment with Nutlin-3, was confirmed.
FIG. 7 shows a graph by which the p53 further overexpression in the senescent cells in the case of the treatment with Nutlin-3, was confirmed.
FIG. 8 shows an image by which the p53 further overexpression in the senescent cells in the case of the treatment with Nutlin-3, was confirmed.
FIG. 9 shows an image by which the induction of cells into senescent cells when the retinal tissue of a mice was treated with doxorubicin, was confirmed by SA-β-gal staining.
FIG. 10 shows an image by which the p53 and p21 overexpression in the senescent cells induced by treating the retinal tissue of a mice with doxorubicin, was confirmed.
FIG. 11 shows an image by which the increase in the drusen marker APOE expression in the senescent cells induced by treating the retinal tissue of a mice with doxorubicin, was confirmed.
FIG. 12 shows an image by which the increase in the amount of drusen and the thickness of the Bruch's membrane due to the treatment of the retinal tissue of a mice with doxorubicin, was confirmed.
FIG. 13 shows an image by which the senescent cell death in the case of the treatment of senescent cells of the retinal tissue of a mice with Nutlin-3, was confirmed.
FIG. 14 shows a graph by which the senescent cell death in the case of the treatment of senescent cells of the retinal tissue of a mice with Nutlin-3, was confirmed.
FIG. 15 shows an image by which the decrease in waste products and the improvement in the cell tissue alignment state in the case of the treatment of senescent cells of the retinal tissue of a mice with Nutlin-3, was confirmed.
FIG. 16 shows an image by which the decrease in the amount of drusen and the thickness of Bruch's membrane in the case of the treatment of senescent cells of the retinal tissue of a mice with Nutlin-3, was confirmed.
FIG. 17 shows a graph by which the decrease in the thickness of Bruch's membrane in the case of the treatment of senescent cells of the retinal tissue of a mice with Nutlin-3, was confirmed.
FIG. 18 shows a graph by which the senescent cell death in the case of the co-treatment of senescent cells of the retinal tissue of a mice with Nutlin-3 and nicotinamide, was confirmed.
FIG. 19 shows an image by which the senescent cell death in the case of the co-treatment of senescent cells of the retinal tissue of a mice with Nutlin-3 and nicotinamide, was confirmed.
FIG. 20 shows a graph by which the decrease in waste products in the case of the co-treatment of senescent cells of the retinal tissue of a mice with Nutlin-3 and nicotinamide, was confirmed.
FIG. 21 shows an image by which the decrease in waste products in the case of the co-treatment of senescent cells of the retinal tissue of a mice with Nutlin-3 and nicotinamide, was confirmed.
FIG. 22 shows an image by which the decrease in drusen marker APOE expression and the improvement in the cell tissue alignment state in the case of co-treatment of the senescent cells of the retinal tissue of a mice with Nutlin-3 and nicotinamide, was confirmed.

### MODE OF DISCLOSURE

Hereinafter, the present disclosure will be described in more detail through Examples. These examples are only intended to show embodiments of the present disclosure, and it would be obvious to those skilled in the art that the scope of the present disclosure would not be interpreted as being limited by these examples.

### Example 1. Confirmation of induction of senescent cells by treatment with doxorubicin

ARPE-19, a human retinal pigment epithelium (RPE) cell line, was induced into senescent cells using doxorubicin (Dox), which is used to form peroxide anions, induce DNA damage, and induce cell aging.

ARPE-19 cells were obtained from American Type Culture Collection (ATCC, CRL-2302, Va., U.S.A.), and cultured using a Dulbecco's modified Eagle's medium/nutrient mixture F-12 (WellGene, LM 002-04, Korea) supplemented with 10% fetal bovine serum (Invitrogen 16000, Waltham, U.S.A.) and penicillin-streptomycin (pen/strep, WellGene, LS 202-02) in a humidifying incubator having 5% CO₂ at the temperature of 37 °C. Cells were used after the cells were subcultured to 15-25 passages and reached the density population of about 70%-about 80%.

The cultured ARPE-19 cells were seeded in 24- or 6-well plates at a density of 25,000 or 100,000 cells per well prior to inducing cell aging with doxorubicin (Dox, Tocris Bioscience # 2252, Bristol, UK). After 24 hours, 250 nM doxorubicin was added and the cells were incubated for another 3 days. After 3 days, the cells were placed in a doxorubicin-free medium and the medium was replaced daily. On day 10, senescent cells (SnCs) that were treated with doxorubicin and non-senescent cells (non-SnCs) that were not treated with doxorubicin but replaced with medium alone were seeded into 24- or 6-well plates for further experiments.

In order to stain ARPE-19 cells which had been grown and aged in a 24-well plate with senescence-associated beta-galactosidase, SA-β-gal, the cells were fixed with a fixing solution at room temperature for 15 minutes, washed with PBS, and stained with a staining solution for 6 hours. Images were captured using an inverted microscope (Carl Zeiss Axio Scope A1, Gottingen, Germany).

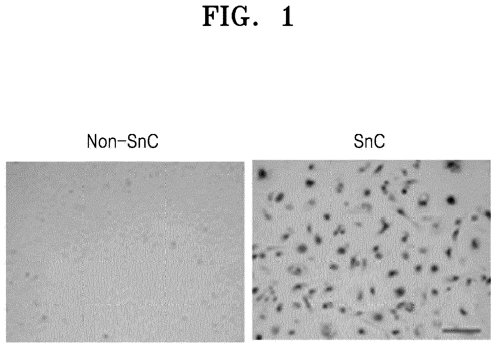

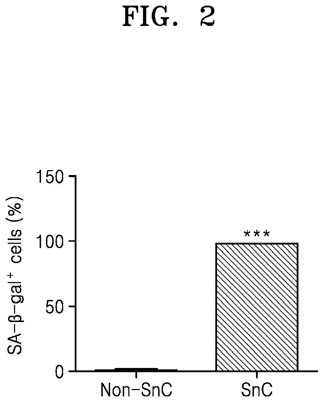

As a result, as shown in FIGS. 1 and 2, it was confirmed that ARPE-19 cells treated with doxorubicin were induced to senescent cells.

### Example 2. Confirmation of p53 and p21 overexpression in senescent cells

In order to confirm whether the senescent cells induced in Example 1 overexpress p53 and p21, an experiment was performed as follows.

Doxorubicin-treated aged cells (SnCs) grown in the 6-well plate of Example 1 and non-aged cells (non-SnCs) were dissolved in RIPA buffer (thermo fisher scientific, 89901) supplemented with protease inhibitor cocktail (Roche, 11697498001, Basel, Switzerland). The protein concentration of cells was quantified using BCA assay (Pierce, 23227, Waltham, USA). The same amount of protein was separated by SDS-PAGE in a 10-12% polyacrylamide gel and transferred to a polyvinylidene difluoride (PVDF) membrane. Then, the PVDF membrane was blocked with 5% skim milk in buffer for 1 hour, and anti-p53 (1: 500, Santa Cruz Biotechnology, SC-126), anti-p21 (1: 500, Santa Cruz Biotechnology, SC-6246) and anti-β-actin (1: 2000, Abcam, ab8226) antibodies were added thereto, incubated overnight at 4 °C, and then incubated with a horseradish peroxidase-conjugated anti-mouse IgG (1: 5000, Cell Signaling, 7076S) antibodies. The immunoreactive proteins were then visualized using a chemiluminescent substrate (Amersham, RDN2232, Little Chalfont, UK) and quantified with a densitometer using ImageJ software (NIH, USA). All experiments were repeated at least three times.

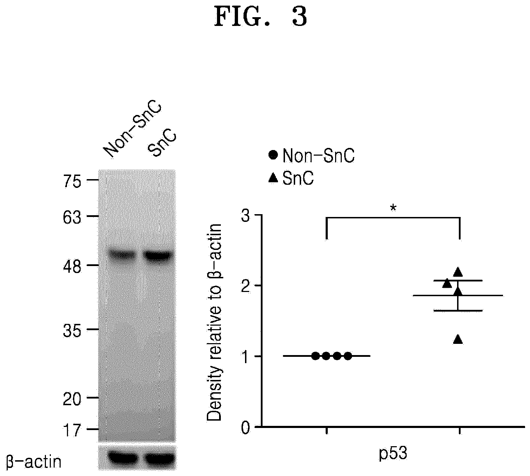

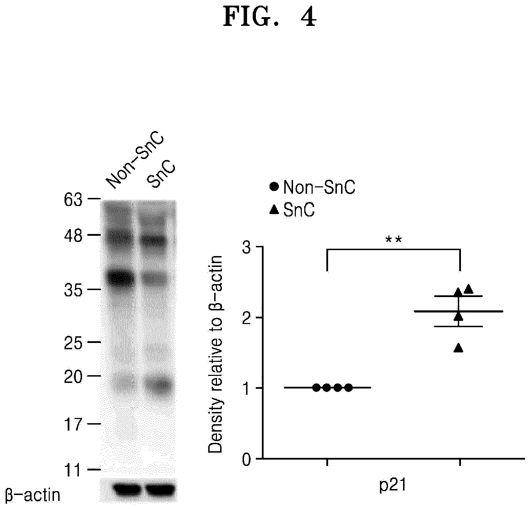

As a result, as shown in FIGS. 3 and 4, it was confirmed that p53 and p21 were more highly expressed in senescent cells than in non-senescent cells.

### Example 3. Confirmation of overexpression of inflammatory factor in senescent cells

In order to confirm whether the senescent cells induced in Example 1 overexpress the inflammatory factor, an experiment was performed as follows.

Total RNA was isolated from doxorubicin-treated aged cells (SnCs) grown in the 6-well plate of Example 1 and non-senescent cells(non-SnCs) by using TRIzol reagent (Invitrogen, 15596026). After RNA isolation, mRNA was transcribed into cDNA using reverse transcriptase (Thermo Fisher Scientific, 4368813). mRNA was quantified with specific primers at 40 cycles using the CFX Connect Real-Time system (BIO-RAD, Calif., USA). The PCR products were identified via melting curve analysis. Relative mRNA expression was calculated using the 2^{-ΔΔCt} method. GAPDH was used as an internal reference gene. The average Ct value was normalized to GAPDH. Real-time PCR was repeated at least three times for each group.

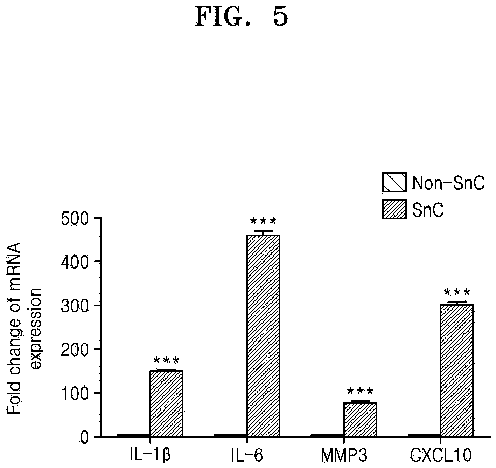

As a result, as shown in FIG. 5, it was confirmed that inflammatory factors were not expressed in non-senescent cells, and IL-1β, IL-6, MMP3, and CXCL10, which are inflammatory factors, were highly expressed in senescent cells.

### Example 4. Confirmation of selective killing effect of Nutlin-3 on senescent cells

To determine whether Nutlin-3 selectively killed senescent cells, the cells were treated with Nutlin-3 as follows and stained with propidium iodide.

Doxorubicin-treated senescent cells (SnCs) grown in the 24-well plate of Example 1 and non-senescent cells (non-SnCs) were treated with Nutlin-3 at different concentrations. After 24 hours, the medium was removed, and a medium including propidium iodide (PI, BD Biosciences, 51-66211E, 1.5 ug/ml) and Hoechst 33342 (Invitrogen, H1399, 1 ug/ml) was added to each well. After 30 minutes, images were obtained using an ultra-high-resolution confocal laser scanning microscope (Carl Zeiss, LSM 800).

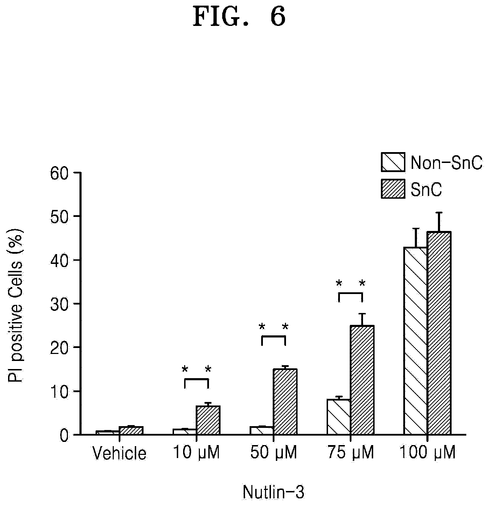

As a result, as shown in FIG. 6, senescent cells were 7 times more killed at 50 uM concentration and 3 times more killed at 75 uM concentration, than non-senescent cells. These results indicate that Nutlin-3 has an effect of selectively killing senescent cells.

### Example 5. Confirmation of p53 overexpression induction effect of Nutlin-3

The following experiment was performed to identify the cause for the selective killing of senescent cells by Nutlin-3 as described in Example 4.

Doxorubicin-treated senescent cells (SnCs) grown in the 6-well plate of Example 1 and non-senescent cells (non-SnCs) were treated with different concentrations of Nutlin-3, and then, dissolved in RIPA buffer (Thermo Fisher Scientific, 89901) supplemented with protease inhibitor cocktail (Roche, 11697498001, Basel, Switzerland). The protein concentration of cells was quantified using BCA assay (Pierce, 23227, Waltham, USA). The same amount of protein was separated by SDS-PAGE in a 10-12% polyacrylamide gel and transferred to a polyvinylidene difluoride (PVDF) membrane. Then, the PVDF membrane was blocked with 5% skim milk powder in buffer for 1 hour, and anti-p53 (1: 500, Santa Cruz Biotechnology, SC-126) and anti-β-actin (1: 2000, Abcam, ab8226) antibodies were added thereto, incubated overnight at 4 °C, and then incubated with horseradish peroxidase-conjugated anti-mouse IgG (1: 5000, Cell Signaling, 7076S) antibodies. The immunoreactive proteins were then visualized using a chemiluminescent substrate (Amersham, RDN2232, Little Chalfont, UK) and quantified with a densitometer using ImageJ software (NIH, USA). All experiments were repeated at least three times.

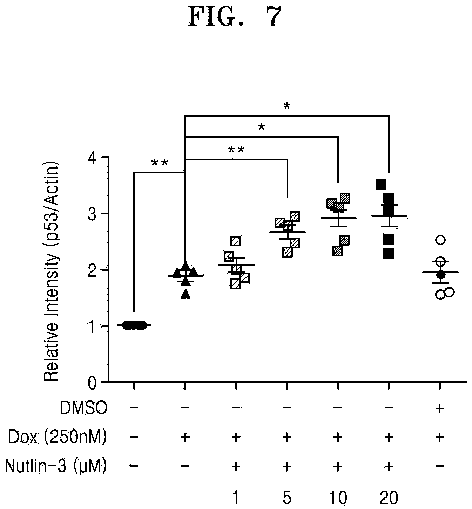

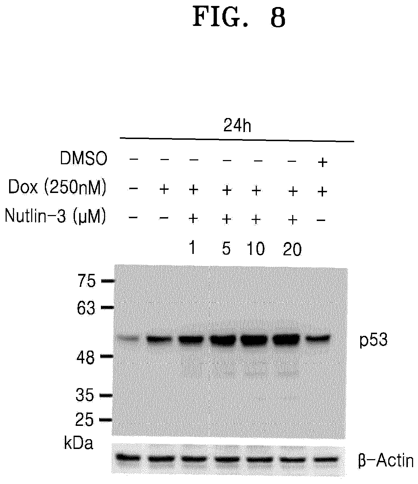

As a result, as shown in FIG. 7 and FIG. 8, the senescent cells were overexpressed compared to the non-senescent cells, and when Nutlin-3 was treated, the expression of p53 was further increased. These results indicate that treatment with Nutlin-3 results in further overexpression of p53, leading to apoptosis of senescent cells.

### Example 6. Determination of induction of senescent cells in vivo

Using a senescent cell inducing substance, the retinal tissue of a mice was induced into senescent cells as follows.

Under an optical microscope (Olympus SZ51, Tokyo, Japan), a small hole was made in the limbus using a 30-gage sterile needle (BD Science, San Jose, USA). Then, a blunt 35-gage Hamilton microsyringe (Hamilton Company, NV, USA) was slowly inserted through the hole. 1 uL of 100 ng/µl Dox or 1.4 µg/µl Alu RNA was injected into the subretinal space of C57BL/6N or C57BL/6J mice for 7 days.

The mice were then anesthetized with CO₂ gas and the eyes were immediately removed therefrom. Anterior eyeballs were dissected in cold PBS. After careful removal of the retina, the RPE/choroid/scleral complex tissues or sectioned retina were immediately fixed with a fixing solution provided together with SA-β-galactosidase (SA-β-gal) staining kit (BioVision, # K320, California, USA) at room temperature for 20 minutes, and stained with a staining solution mix provided together with the SA-β-gal staining kit according to the manufacturer's protocol. After SA-β-gal staining overnight at 37 °C, the dark melanin pigment of the RPE/choroid was bleached to show SA-β-gal staining obscured by the melanin pigment in these tissues. For the decolorization of RPE/choroid, the RPE/choroid/sclera complex tissue was immersed in 30% H₂O₂, incubated for 45 minutes at a heat block of 55 °C, then rinsed with PBS and fixed flat with a micro-point pincer and a surgical blade (World Precision Instruments, Florida, USA) under an optical microscope (Olympus SZ51). Images of stained RPE/choroid flat mounts or sectioned retina were captured using an inverted microscope (Leica Microsystems # DMi1, Wetzlar, Germany).

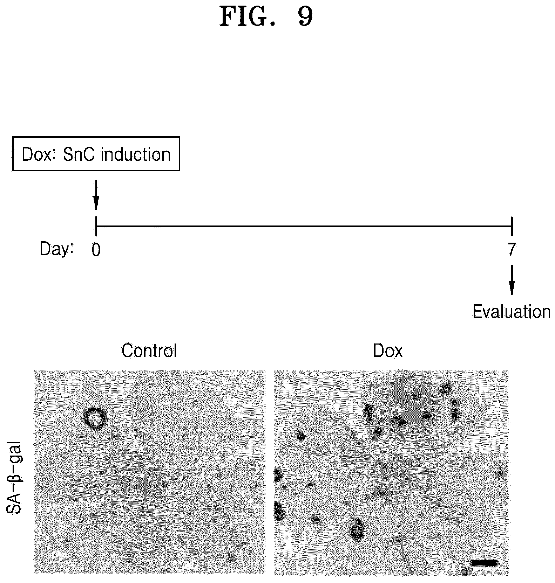

As a result, as shown in FIG. 9, it was confirmed that cells of the retina tissue of the mice were induced into senescent cells.

### Example 7. Confirmation of p53 and p21 overexpression of senescent cells in vivo

In order to confirm whether the senescent cells induced in Example 6 overexpress p53 and p21, an experiment was performed as follows.

After blocking the retinal tissue with 1% BSA in PBS for 1 hour, the fixed tissue or cells were incubated overnight at 4 °C with primary antibodies against p53 (1:50, Santa Cruz Biotechnology, Texas, USA) and p21 (1:50, Santa Cruz Biotechnology). Stained tissues or cells were washed twice with PBS for 5 minutes and incubated with Alexa Fluor-conjugated secondary antibody for 2 hours at room temperature. As the secondary antibody, Alexa Fluor 555-conjugated goat anti-mouse IgG (Thermo Fisher Scientific, A-11029, Waltham, USA), Alexa Fluor 488-conjugated goat anti-mouse IgG (Thermo Fisher Scientific, A-21424), Alexa Fluor 555-conjugated goat anti-rabbit IgG (Thermo Fisher Scientific, A-21424), or Alexa Fluor 488-conjugated \u200b\u200b goat anti-rabbit IgG (Thermo Fisher Scientific, A-11034) was used. All secondary antibodies were used after being diluted at the ratio of 1:1000. After incubation with the secondary antibody, tissues or cells were stained with nuclear dye DAPI (1:4000, Thermo Fisher Scientific, 62248) or Hoechst 33342 (1:3000, Thermo Fisher Scientific, H3570) in PBS for 15 minutes at room temperature. Thereafter, the tissues were then fixed in a fixing media (Ployscience, 18606-20, PA, USA). Stained cells were observed with an inverted microscope (Carl Zeiss, LSM 900, Oberkochen, Germany).

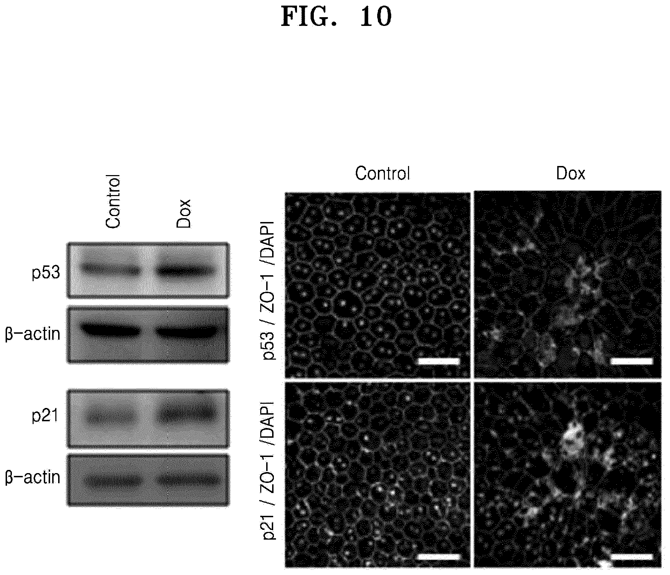

As a result, as shown in FIG. 10, it was confirmed that, compared to normal cells which were not induced to senescent cells, cells of retinal tissue of a mice induced to senescent cells overexpressed p53 and p21.

### Example 8. Confirmation of drusen expression by senescent cells in vivo

In order to confirm the appearance of drusen by senescent cells induced in Example 6, an experiment was performed as follows.

After blocking the retinal tissue with 1% BSA in PBS for 1 hour, the fixed tissue or cells were incubated with primary antibody (1:50, Santa Cruz Biotechnology) against APOE, a drusen marker, overnight at 4 °C. Stained tissues or cells were washed twice with PBS for 5 minutes and incubated with Alexa Fluor-conjugated secondary antibody for 2 hours at room temperature. As the secondary antibody, Alexa Fluor 555-conjugated goat anti-mouse IgG (Thermo Fisher Scientific, A-11029, Waltham, USA), Alexa Fluor 488-conjugated goat anti-mouse IgG (Thermo Fisher Scientific, A-21424), Alexa Fluor 555-conjugated goat anti-rabbit IgG (Thermo Fisher Scientific, A-21424), or Alexa Fluor 488-conjugated \u200b\u200b goat anti-rabbit IgG (Thermo Fisher Scientific, A-11034) was used. All secondary antibodies were used after being diluted at the ratio of 1:1000. After incubation with the secondary antibody, tissues or cells were stained with nuclear dye DAPI (1:4000, Thermo Fisher Scientific, 62248) or Hoechst 33342 (1:3000, Thermo Fisher Scientific, H3570) in PBS for 15 minutes at room temperature. Thereafter, the tissues were then fixed in a fixing media (Ployscience, 18606-20, PA, USA). Stained cells were observed with an inverted microscope (Carl Zeiss, LSM 900, Oberkochen, Germany).

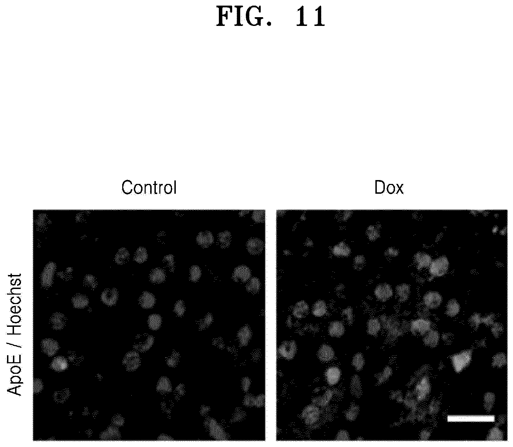

As a result, as shown in FIG. 11, it was confirmed that, compared to normal cells which were not induced to senescent cells, cells of retinal tissue of a mice induced to senescent cells expressed drusen.

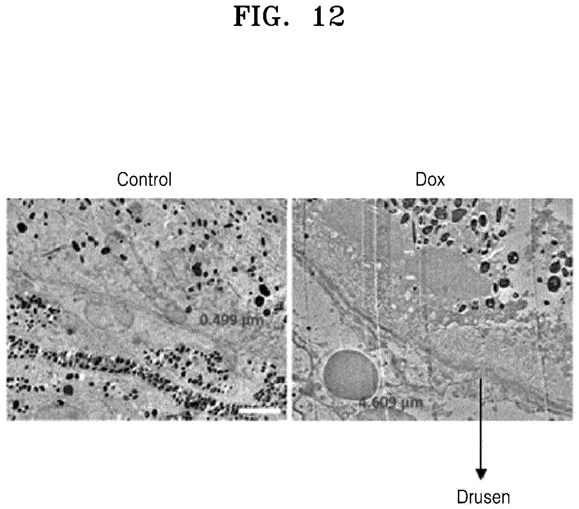

In addition, the eye of mice was fixed with a solution including 2% glutaraldehyde and 3.75% sucrose in 0.1 M sodium phosphate buffer (pH 7.0), for 30 minutes, and washed three times with 0.1M PBS including 3.75% sucrose for 30 minutes to prepare a sample for TEM. The eye was fixed with a solution including 0.1% osmium tetraoxide and 5% sucrose in 0.05 M sodium phosphate buffer (pH 7.0) for 1 hour and rinsed three times with distilled water. The sample was then dehydrated in a series of acetone solutions (step 20%), which were graded, and embedded in an epoxy resin. The resin was polymerized at 70 °C for 12 hours. Ultra-thin sections (100 nm) were obtained using RMC CR-X ultra microtomes and then images thereof were obtained using a JEOL JEM-1400 electron microscope. The thickness of the Bruch's membrane was measured using TEM. The average thickness of the Bruch's film was determined by measuring the thicknesses in the nine images of respective groups, and the results are shown in FIG. 12. As shown in FIG. 12, it was confirmed that when senescent cells were induced by doxorubicin, the amount of drusen was increased and the thickness of the Burk layer was increased.

### Example 9. Confirmation of Senescent Cell Killing Effect by Nutlin-3 in vivo

Under an optical microscope (Olympus SZ51, Tokyo, Japan), a small hole was made in the limbus using a 30-gage sterile needle (BD Science, San Jose, USA). Then, a blunt 35-gage Hamilton microsyringe (Hamilton Company, NV, USA) was slowly inserted through the hole. After injecting 100 ng/µl Dox or 1 uL of 1.4 µg/µl Alu RNA into the subretinal space of C57BL/6N or C57BL/6J mice for 7 days, 20 ng/µl Nutlin-3 (# SML0580, St. Louis , USA) or vehicle (0.4% DMSO in normal saline) was injected once every 3 days for 7 days, for a total of 2 intravitreal injections, with a blunt 35-gauge Hamilton microsyringe.

The mice were then anesthetized with CO₂ gas and the eyes immediately removed therefrom. Anterior eyeballs were dissected in cold PBS. After careful removal of the retina, the RPE/choroid/scleral complex tissues or sectioned retina were immediately fixed with a fixing solution provided together with SA-β-galactosidase (SA-β-gal) staining kit (BioVision, # K320, California, USA) at room temperature for 20 minutes, and stained with a staining solution mix provided together with the SA-β-gal staining kit according to the manufacturer's protocol. After SA-β-gal staining overnight at 37 °C, the dark melanin pigment of the RPE/choroid was bleached to show SA-β-gal staining obscured by the melanin pigment in these tissues. For the decolorization of RPE/choroid, the RPE/choroid/sclera complex tissue was immersed in 30% H₂O₂, incubated for 45 minutes at a heat block of 55 °C, then rinsed with PBS and fixed flat with a micro-point pincer and a surgical blade (World Precision Instruments, Florida, USA) under an optical microscope (Olympus SZ51). Images of stained RPE/choroid flat mounts or sectioned retina were captured using an inverted microscope (Leica Microsystems # DMi1, Wetzlar, Germany).

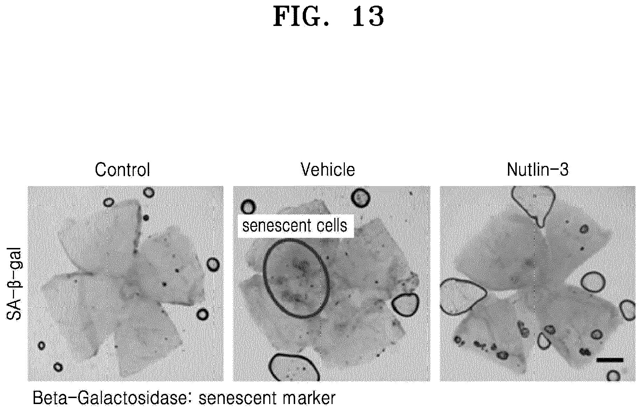

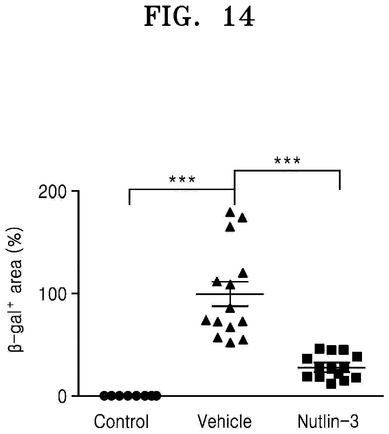

As a result, as shown in FIG. 13 and FIG. 14, compared to the group injected with a vehicle, it was confirmed that β-galactosidase expression was low in the group injected with Nutlin-3, which indicates that senescent cell death has occurred. These results indicate that Nutlin-3 has an effect of killing senescent cells.

### Example 10. Confirmation of lesion mitigation effect by Nutlin-3 in vivo

After inducing senescent cells in Example 9, a lesion state of an eye injected with Nutlin-3 or vehicle was identified as follows.

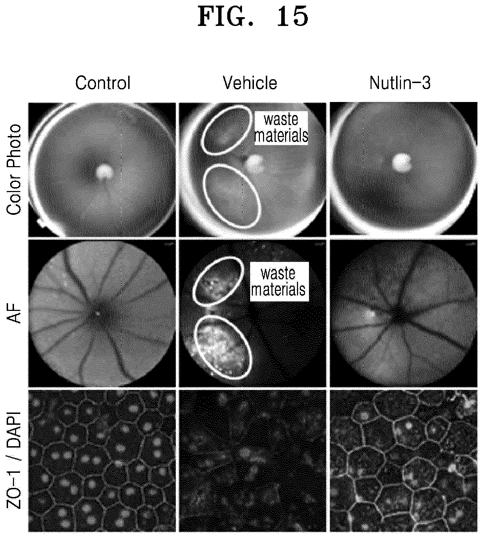

After dilation of the eye with TRC-50 IX camera (Topcon, Tokyo, Japan) connected to a digital imaging system (Nikon, Japan) and HRA2 (Heidelberg Engineering, Heidelberg, Germany), the color fundus image and fundus autofluorescence image of the eye of the mice were each captured. As a result, as shown in FIG. 15, it was confirmed that the waste was significantly reduced in the group injected with Nutlin-3, compared to the group injected with the vehicle with waste.

In addition, in order to identify the cell tissue alignment state, ZO-1 expression was confirmed. Specifically, after blocking the retinal tissue with 1% BSA in PBS for 1 hour, fixed tissue or cells were incubated with primary antibody against ZO-1 (1:250, Invitrogen) overnight at 4 °C. Stained tissues or cells were washed twice with PBS for 5 minutes and incubated with Alexa Fluor-conjugated secondary antibody for 2 hours at room temperature. As the secondary antibody, Alexa Fluor 555-conjugated goat anti-mouse IgG (Thermo Fisher Scientific, A-11029, Waltham, USA), Alexa Fluor 488-conjugated goat anti-mouse IgG (Thermo Fisher Scientific, A-21424), Alexa Fluor 555-conjugated goat anti-rabbit IgG (Thermo Fisher Scientific, A-21424), or Alexa Fluor 488-conjugated \u200b\u200b goat anti-rabbit IgG (Thermo Fisher Scientific, A-11034) was used. All secondary antibodies were used after being diluted at the ratio of 1:1000. After incubation with the secondary antibody, tissues or cells were stained with nuclear dye DAPI (1:4000, Thermo Fisher Scientific, 62248) or Hoechst 33342 (1:3000, Thermo Fisher Scientific, H3570) in PBS for 15 minutes at room temperature. Thereafter, the tissues were then fixed in a fixing media (Ployscience, 18606-20, PA, USA). Stained cells were observed with an inverted microscope (Carl Zeiss, LSM 900, Oberkochen, Germany). As a result, as shown in FIG. 15, it was confirmed that the group injected with Nutlin-3 showed improved cell tissue alignment compared to the group injected with a vehicle.

The results indicate that senescent cell death by Nutlin-3 lead to the decrease in the waste of retinal tissue and the improvement in the cell tissue alignment, and thus, the lesion was alleviated.

### Example 11. Confirmation of drusen expression inhibition effect by Nutlin-3 in vivo

After inducing senescent cells in Example 9, a lesion state of an eye injected with Nutlin-3 or vehicle was identified as follows.

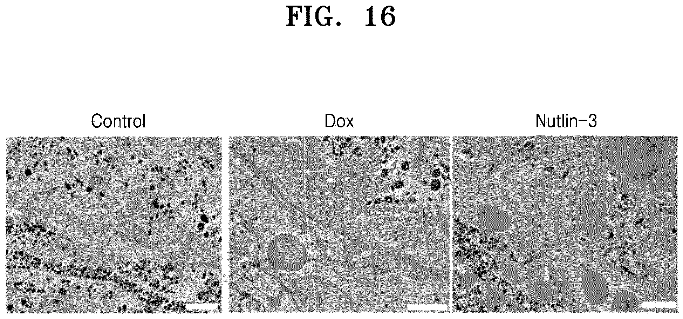

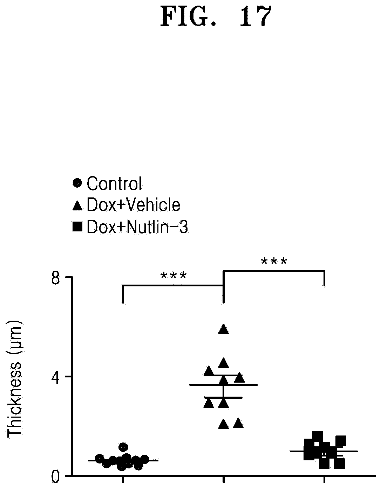

The eyeball of mice in a group injected with a vehicle alone as in Example 9, a group injected with doxorubicin and a vehicle, and a group injected with doxorubicin and Nutlin-3, were fixed with a solution including 2% glutaraldehyde and 3.75% sucrose in 0.1M sodium phosphate buffer (pH 7.0) for 30 minutes, and washed three times with 0.1M PBS including 3.75% sucrose for 30 minutes to prepare a sample for TEM. The eye was fixed with a solution including 0.1% osmium tetraoxide and 5% sucrose in 0.05 M sodium phosphate buffer (pH 7.0) for 1 hour and rinsed three times with distilled water. The sample was then dehydrated in a series of acetone solutions (step 20%), which are graded, and embedded in an epoxy resin. The resin was polymerized at 70 °C for 12 hours. Ultra-thin sections (100 nm) were obtained using RMC CR-X ultra microtomes and then images thereof were obtained using a JEOL JEM-1400 electron microscope. The thickness of the Bruch's membrane was measured using TEM. The average thickness of the Bruch's film was determined by measuring the thicknesses in the nine images of respective groups, and the results are shown in FIGS. 16 and 17.

As shown in FIGS. 16 and FIG. 17, it was confirmed that when Nutlin-3 was administered, the amount of drusen was reduced and the thickness of the Bruch's membrane was reduced, compared to the vehicle-only senescent cells. The results indicate that the drusen of the retinal tissue was reduced due to the senescent cell death by Nutlin-3, and thus the lesion was alleviated.

### Example 12. Confirmation of senescent cell killing effect when administered with combination of Nutlin-3 and nicotinamide in vivo

Under an optical microscope (Olympus SZ51, Tokyo, Japan), a small hole was made in the limbus using a 30-gage sterile needle (BD Science, San Jose, USA). Then, a blunt 35-gage Hamilton microsyringe (Hamilton Company, NV, USA) was slowly inserted through the hole. After injecting 1 uL of 100 ng/µl Dox or 1.4 µg/µl Alu RNA into the subretinal space of C57BL/6N or C57BL/6J mice for 7 days, 20 ng/µl Nutlin-3 (# SML0580, St. Louis , USA) or vehicle (0.4% DMSO in normal saline) was administered to the retina on day 0 and day 3, and 50 mg/kg of Nutlin-3 and 100 mg/kg of nicotinamide were mixed at the ratio of 1:2 and the mixture was administered orally through the feeding needle catheter every day for 3 days.

The mice were then anesthetized with CO₂ gas and the eyes immediately removed therefrom. Anterior eyeballs were dissected in cold PBS. After careful removal of the retina, the RPE/choroid/scleral complex tissues or sectioned retina were immediately fixed with a fixing solution provided together with SA-β-galactosidase (SA-β-gal) staining kit (BioVision, # K320, California, USA) at room temperature for 20 minutes, and stained with a staining solution mix provided together with the SA-β-gal staining kit according to the manufacturer's protocol. After SA-β-gal staining overnight at 37 °C, the dark melanin pigment of the RPE/choroid was bleached to show SA-β-gal staining obscured by the melanin pigment in these tissues. For the decolorization of RPE/choroid, the RPE/choroid/sclera complex tissue was immersed in 30% H₂O₂, incubated for 45 minutes at a heat block of 55 °C, then rinsed with PBS and fixed flat with a micro-point pincer and a surgical blade (World Precision Instruments, Florida, USA) under an optical microscope (Olympus SZ51). Images of stained RPE/choroid flat mounts or sectioned retina were captured using an inverted microscope (Leica Microsystems # DMi1, Wetzlar, Germany).

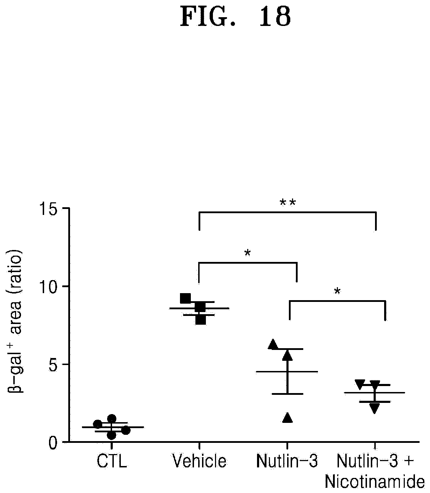

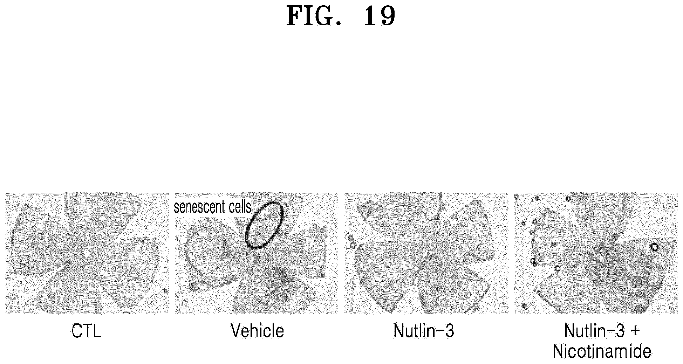

As a result, as shown in FIG. 18 and FIG. 19, it was confirmed that senescent cells of retinal tissue were more killed in the group administered with the combination of Nutlin-3 and nicotinamide.

### Example 13. Confirmation of lesion alleviation effect when administered with combination of Nutlin-3 and nicotinamide in vivo

After inducing senescent cells in Example 12, the lesion state of the eye injected with Nutlin-3 alone or the combination of Nutlin-3 and nicotinamide, was confirmed as follows.

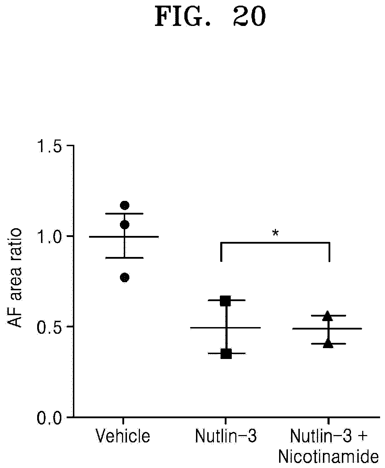

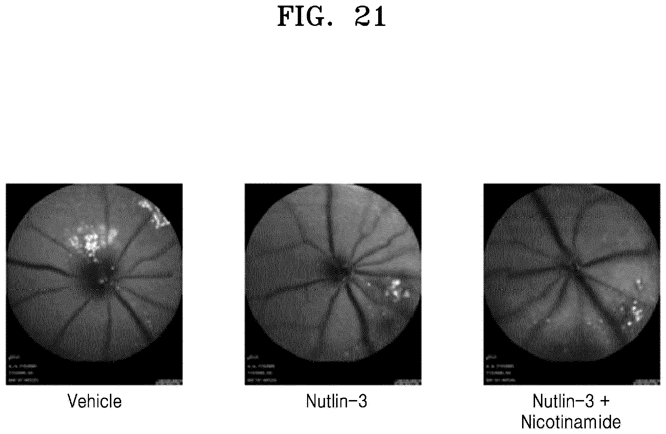

After dilation of the eye with TRC-50 IX camera (Topcon, Tokyo, Japan) connected to a digital imaging system (Nikon, Japan) and HRA2 (Heidelberg Engineering, Heidelberg, Germany), the fundus autofluorescence image of the eye of the mice was captured. As a result, as shown in FIG. 20 and FIG. 21, it was confirmed that the waste was significantly reduced in the group to which Nutlin-3 or the combination of Nutlin-3 and nicotinamide was injected, compared to the group to which a vehicle having waste was injected, and it was confirmed that the group to which the combination of Nutlin-3 and nicotinamide was administered had a smaller error range than the group to which Nutlin-3 alone was injected.

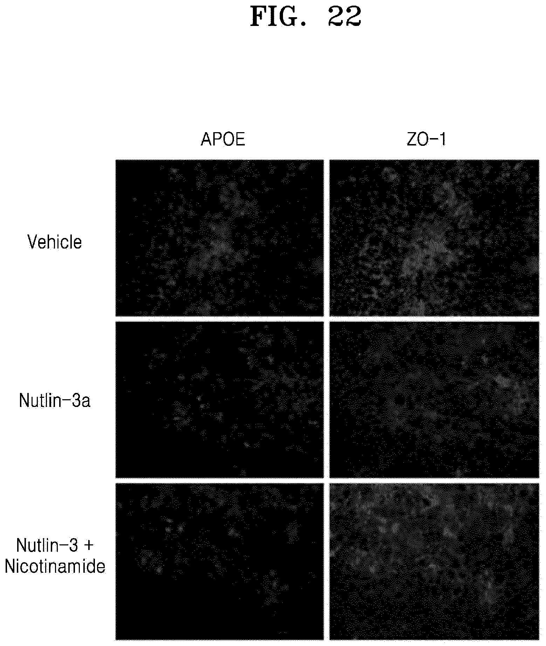

In addition, the expression of ZO-1 and the expression of the drusen marker APOE were confirmed to identify the cell tissue alignment state. Specifically, after blocking the retinal tissue with 1% BSA in PBS for 1 hour, fixed tissue or cells were incubated with primary antibody (1:50, Santa Cruz Biotechnology) against ZO-1 (1:250, Invitrogen) or APOE overnight at 4 °C. Stained tissues or cells were washed twice with PBS for 5 minutes and incubated with Alexa Fluor-conjugated secondary antibody for 2 hours at room temperature. As the secondary antibody, Alexa Fluor 555-conjugated goat anti-mouse IgG (Thermo Fisher Scientific, A-11029, Waltham, USA), Alexa Fluor 488-conjugated goat anti-mouse IgG (Thermo Fisher Scientific, A-21424), Alexa Fluor 555-conjugated goat anti-rabbit IgG (Thermo Fisher Scientific, A-21424), or Alexa Fluor 488-conjugated \u200b\u200b goat anti-rabbit IgG (Thermo Fisher Scientific, A-11034) was used. All secondary antibodies were used after being diluted at the ratio of 1:1000. After incubation with the secondary antibody, tissues or cells were stained with nuclear dye DAPI (1:4000, Thermo Fisher Scientific, 62248) or Hoechst 33342 (1:3000, Thermo Fisher Scientific, H3570) in PBS for 15 minutes at room temperature. Thereafter, the tissues were then fixed in a fixing media (Ployscience, 18606-20, PA, USA). Stained cells were observed with an inverted microscope (Carl Zeiss, LSM 900, Oberkochen, Germany). As a result, as shown in FIG. 22, it was confirmed that compared to the group injected with a vehicle, the group injected with Nutlin-3 or the combination of Nutlin-3 and nicotinamide showed the improved cell tissue alignment state. In addition, it was confirmed that the group injected with the combination of Nutlin-3 and nicotinamide showed smaller drusen and better cell tissue alignment state compared to the group administered with Nutlin-3 alone.

## Claims

1. A pharmaceutical composition for preventing or treating macular degeneration, the pharmaceutical composition comprising, as an active ingredient, an imidazoline derivative compound represented by Formula 1 or a pharmaceutically acceptable salt thereof: 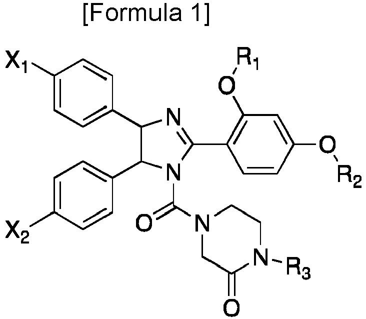 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

2. The pharmaceutical composition of claim 1, wherein the imidazoline derivative compound is Nutlin-1, Nutlin-2, or Nutlin-3.

3. The pharmaceutical composition of claim 1, further comprising an antioxidant.

4. The pharmaceutical composition of claim 3, wherein the antioxidant comprises at least one selected from nicotinamide, anthocyanin, benzenediol abietane diterpene, carnosine, carotenoid, xanthophyll, carotenoid in saffran, curcuminoid, cyclopentenone prostaglandin, flavonoid, prenylflavonoid, retinoid, stilbenoid, uric acid, vitamin A, vitamin B1, vitamin B2, vitamin B3, vitamin B6, vitamin B9, vitamin B12, vitamin C, vitamin E, selenium, zinc, an inhibitor and scavenger of lipid peroxidation and by-products thereof, tirilazad, and analogs, derivatives, salts, and combinations thereof.

5. The pharmaceutical composition of claim 1, wherein the imidazoline derivative compound overexpresses at least one of p53 and p21 in senescent cells.

6. The pharmaceutical composition of claim 1, wherein the macular degeneration comprises one or more selected from age-related macular degeneration (AMD), wet macular degeneration, and dry macular degeneration.

7. A pharmaceutical composition for preventing or treating macular degeneration, the pharmaceutical composition comprising, as an active ingredient, an imidazoline derivative compound represented by Formula 1 or a pharmaceutically acceptable salt thereof, wherein the imidazoline derivative compound is co-administered with an antioxidant: 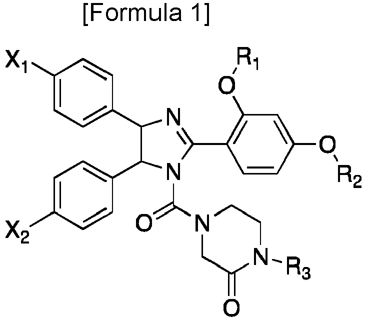 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

8. The pharmaceutical composition of claim 7, wherein the co-administration is performed by simultaneously administering the imidazoline derivative compound and the antioxidant, or by administering any one of the imidazoline derivative compound and the antioxidant and then the other one.

9. A health functional food for preventing or ameliorating macular degeneration, the health functional food comprising, as an active ingredient, an imidazoline derivative compound represented by Formula 1 or a salt thereof: 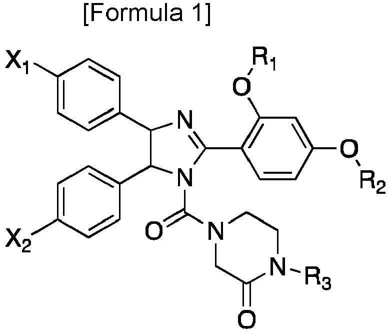 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

10. The health functional food of claim 9, further comprising an antioxidant.

11. A method of preventing or treating macular degeneration, the method comprising administering an imidazoline derivative compound represented by Formula 1 or a pharmaceutically acceptable salt thereof to a subject in need thereof: 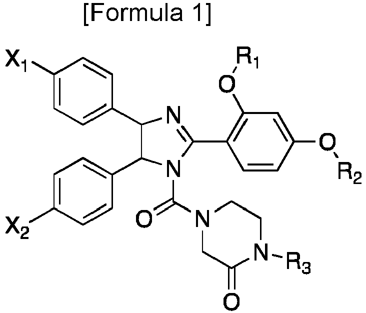 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.

12. Use of an imidazoline derivative compound represented by Formula 1 or pharmaceutically acceptable salt thereof for preparing a composition for preventing or treating macular degeneration: 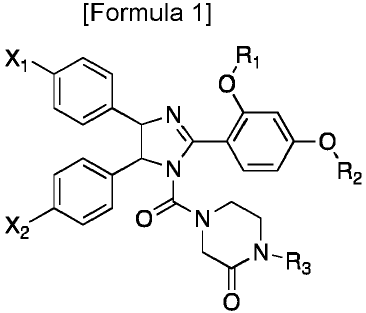 wherein R₁ to R₃ are each independently H, a C₁ to C₁₀ alkyl, a C₂ to C₁₀ alkenyl, a C₂ to C₁₀ alkynyl, or a C₁ to C₁₀ alkoxy, and X₁ and X₂ are each independently halogen.
